(19)

**Europäisches Patentamt**
**European Patent Office**
**Office européen des brevets**

(11)  **EP 3 673 254 B1**

(12)  # EUROPEAN PATENT SPECIFICATION

(45) Date of publication and mention
of the grant of the patent:
**07.06.2023  Bulletin 2023/23**

(21) Application number: **18756207.9**

(22) Date of filing: **15.08.2018**

(51) International Patent Classification (IPC):
**G01N 15/02** (2006.01)      **G01N 15/14** (2006.01)
**G01N 33/49** (2006.01)      **G01N 15/00** (2006.01)
**G01N 15/10** (2006.01)

(52) Cooperative Patent Classification (CPC):
**G01N 15/0227; G01N 15/1475; G01N 33/5094;**
G01N 2015/0073; G01N 2015/1006;
G01N 2015/1486; G01N 2015/1493

(86) International application number:
**PCT/EP2018/072127**

(87) International publication number:
**WO 2019/038155 (28.02.2019 Gazette 2019/09)**

(54)  **CLASSIFICATION, SCREENING AND DIAGNOSTIC METHODS AND APPARATUS**

VERFAHREN UND VORRICHTUNG FÜR KLASSIFIZIERUNG, SCREENING UND DIAGNOSE

PROCÉDÉS ET APPAREIL DE CLASSIFICATION, CRIBLAGE ET DIAGNOSTIC

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **23.08.2017  GB 201713568**

(43) Date of publication of application:
**01.07.2020  Bulletin 2020/27**

(73) Proprietors:
• **Epigem Limited**
**Redcar, Yorkshire TS10 5SQ (GB)**
• **University of Zürich**
**8006 Zürich (CH)**

(72) Inventors:
• **RYAN, Timothy George**
**Middlesborough, Great Ayton TS9 6HP (GB)**
• **MAKHRO, Asya**
**8057 Zurich (CH)**

(74) Representative: **Suddaby, Mark Edward**
**Novagraaf UK**
**3rd Floor**
**77 Gracechurch Street**
**London EC3V 0AS (GB)**

(56) References cited:
• **AMIREAULT PASCAL ET AL: "2343: Contribution of Imaging Flow Cytometry to Storage Lesion Assessment: Identification of a Sub-Population of Morphologically Altered Erythrocytes", BLOOD; 57TH ANNUAL MEETING OF THE AMERICAN-SOCIETY-OF-HEMATOLOGY, AMERICAN SOCIETY OF HEMATOLOGY, US; ORLANDO, FL, USA , vol. 126, no. 23 3 December 2015 (2015-12-03), page 2343, XP009508743, ISSN: 0006-4971 Retrieved from the Internet: URL:http://www.bloodjournal.org/content/12 6/23/2343**
• **INNOCENT SAFEUKUI ET AL: "Surface Area Loss and Increased Sphericity Account for the Splenic Entrapment of Subpopulations of Plasmodium falciparum Ring-Infected Erythrocytes", PLOS ONE, vol. 8, no. 3, 1 March 2013 (2013-03-01), page e60150, XP055516620, DOI: 10.1371/journal.pone.0060150**

EP 3 673 254 B1

**(Cont. next page)**

- JAFERZADEH KEYVAN ET AL: "Quantitative investigation of red blood cell three-dimensional geometric and chemical changes in the storage lesion using digital holographic microscopy", INTERNATIONAL SOCIETY FOR OPTICAL ENGINEERING, SPIE, PO BOX 10 BELLINGHAM WA 98227-0010 USA, vol. 20, no. 11, 1 November 2015 (2015-11-01), page 111218, XP060071839, ISSN: 1083-3668, DOI: 10.1117/1.JBO.20.11.111218 [retrieved on 2015-10-21]
- HOA V. PHAM ET AL: "Real Time Blood Testing Using Quantitative Phase Imaging", PLOS ONE, vol. 8, no. 2, 6 February 2013 (2013-02-06), page e55676, XP055516622, DOI: 10.1371/journal.pone.0055676

**Description**

Field of the Invention

The invention relates to methods and apparatus for monitoring the physiological state of red blood cells (RBCs). This provides methods for detecting abnormalities in such cells, giving the ability to pre-screen patients for such abnormalities (such as those produced by anaemias, especially rare anaemias). Methods are also presented for diagnosing such diseases. Apparatus for carrying out such methods are also disclosed.

Background and Prior Art

It is known that red blood cells can adopt a number of morphological forms, and that the presence of some of these forms can be indicative of a disease state.

Automated blood cell analysers are able to provide counts of red and white blood cells, and of platelets. Centrifugal segregation of red blood cells in a density gradient gives an indication of the presence and relative abundance of the various morphological forms of the red blood cells but requires 0.5-1.0 ml of blood, limiting the use of this technique to adult patients. However, accurate identification of the distribution of morphological forms of RBCs typically requires time-consuming and expensive analysis by direct visual inspection of cells by a skilled technician. Blood films (smears) that are currently used for assessing RBC morphology do not always adequately reflect true morphological appearance of cells in suspension.

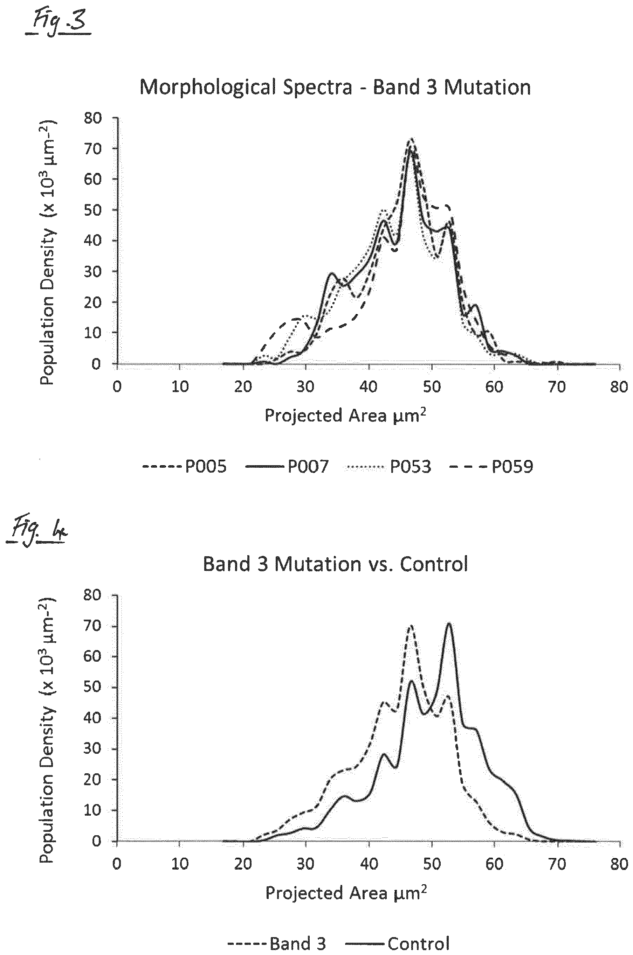

Some recent advances (Deb, N. and Chakroborty, S., "A Noble Technique for Detecting Anemia through Classification of Red Blood Cells in a Blood Smear", IEEE International Conference on Recent Advances and Innovations in Engineering (ICRAIE-2014), May 09-11, Jaipur, India. Fig 3 of that paper shows that a lot of shape artefacts may be introduced by this technique: cells are "angular" and artificially elongated in one direction, also "echinocytes" may appear as a result of film drying. The authors show that analysis of images of RBCs involving extraction of colour data from images, measurement of cell aspect ratios and the calculation of Fourier descriptors for blood cells (involving the calculation of the spatial frequency content of the boundary of the cells) can be used to provide at least a degree of differentiation between different RBC morphologies and differentiation between white blood cells and nucleated RBCs.

Despite these advances, automatic morphological analysis of RBCs for use in diagnostic tests, pre-screening and patient monitoring is not yet available.

For many anaemias, especially the rare anaemias, genetic analysis to identify individual mutations or groups of mutations is required to reach even a putative diagnosis.

AMIREAULT PASCAL ET AL, "2343: Contribution of Imaging Flow Cytometry to Storage Lesion Assessment: Identification of a Sub-Population of Morphologically Altered Erythrocytes", vol. 126, no. 23, ISSN 0006-4971, (20151203), page 2343, BLOOD; 57TH ANNUAL MEETING OF THE AMERICAN-SOCIETY-OF-HEMATOLOGY, AMERICAN SOCIETY OF HEMATOLOGY, US; ORLANDO, FL, USA discloses characterizing the morphological alteration of stored erythrocytes using imaging flow cytometry. Morphological, biochemical, metabolic, and bio-mechanical characteristics of erythrocytes stored in optimal blood bank conditions were analysed. Parameters such as mean corpuscular volume, intracellular ATP level, hemolysis, osmotic fragility, deformability and the plasma levels of ions and metabolites were studied, and a "projected surface area" distribution on normalized frequency plots was developed.

INNOCENT SAFEUKUI ET AL, "Surface Area Loss and Increased Sphericity Account for the Splenic Entrapment of Subpopulations of Plasmodium falciparum Ring-Infected Erythrocytes", PLOS ONE, (20130301), vol. 8, no. 3 discloses the use of imaging technology to analyze infected RBCs' morphology and cell dimensions before and after fractionation with microsphiltration to better understand the changes in the mechanical properties of red blood cells (RBCs) following invasion by the malaria parasite, Plasmodium falciparum.

JAFERZADEH KEYVAN ET AL, "Quantitative investigation of red blood cell three-dimensional geometric and chemical changes in the storage lesion using digital holographic microscopy", INTERNATIONAL SOCIETY FOR OPTICAL ENGINEERING, SPIE, PO BOX 10 BELLINGHAM WA 98227-0010 USA, vol. 20, no. 11 quantitatively investigates RBC 3-D geometric changes in the storage lesion using digital holographic microscopy. Experimental results show the substantial geometric transformation of the biconcave-shaped RBCs to the spherocyte occurs due to RBC storage lesion. Quantitative analysis further showed there are significant correlations between chemical and morphological properties of RBCs.

HOA V. PHAM ET AL, "Real Time Blood Testing Using Quantitative Phase Imaging", PLOS ONE, (20130206), vol. 8, no. 2 demonstrates a real-time blood testing system providing remote diagnosis using quantitative phase imaging for extracting red blood cell morphology. A variety of diagnostic parameters were measured from each cell (e.g., surface area, sphericity, and minimum cylindrical diameter).

It is among the objects of the present invention to attempt a solution to these and other problems.

Summary of the Invention

Accordingly, the invention provides a method of measuring the morphological profile of a sample of red blood cells (RBCs) comprising the steps of: (a) measuring the greatest projected area of each RBC in a population of said RBCs , wherein the greatest projected area of each RBC is measured by:

(i) allowing said RBCs to sediment onto a surface; and
(ii) measuring the projected surface area of each RBC in a direction substantially normal to the plane of said surface;
(b) creating a projected area distribution of the RBCs from said measurements; and (c) identifying characteristic peaks from said projected area distribution;

each characteristic peak having an associated population density and corresponding with a projected area, the population densities of the characteristic peaks indicating the proportion of phenotypic cell types within the RBC sample.

Preferably, said characteristic peaks are those peaks located closest to the following projected areas: Peak 1: 25 $\mu m^2$; Peak 2: 30 $\mu m^2$; Peak 3: 36 $\mu m^2$;Peak 4: 42 $\mu m^2$; Peak 5: 46 $\mu m^2$ ; Peak 6: 53 $\mu m^2$; Peak 7: 57 $\mu m^2$; Peak 8: 63 $\mu m^2$, and wherein each peak indicates the following RBC morphology; Peak 1: terminal echinocytosis; Peak 2: echinocytosis; Peak 3: stomato-RBC; Peak 4: stomato-biconcave; Peak 5: tense biconcave; Peak 6: common biconcave; Peak 7: wavy relaxed biconcave; and Peak 8: relaxed biconcave.

The invention also provides a method of pre-screening for a disorder affecting red blood cells (RBCs) in a subject comprising the steps of: (a) providing a sample of red blood cells previously obtained from a subject; (b) measuring the greatest projected area of each RBC in a population of said RBCs, wherein the greatest projected area of each RBC is measured by:

(i) allowing said RBCs to sediment onto a surface; and
(ii) measuring the projected surface area of each RBC in a direction substantially normal to the plane of said surface;

(c) creating a projected area distribution of the RBCs from said measurements; (d) providing a database storing like projected area distributions of RBCs obtained from subjects including those with a range of such disorders; and (e) comparing said measured projected area distribution to those stored in said database to find a closest match, the potential RBC disorder being that associated with the matched distribution.

Preferably said disorder is a red blood cell disorder, and more preferably, an anaemia.

Preferably, said anaemia is an anaemia selected from the group consisting of: Aceruloplasminemia ; Adenosine deaminase increased activity (ADA) ; Adenylate kinase deficiency ; Aldolase deficiency ; Alpha-thalassaemia - trait or carrier; Aplastic anemia ; Atransferrinemia ; Atypical hemolytic uremic syndrome ; Autoimmune Haemolyitic Anaemia ; Beta-thalassaemia - trait or carrier; Beta-thalassaemia major (and intermedia) ; CDA with thrombocytopenia (GATA I mutation) ; Compound heterozygous sickling disorders ; Congenital acanthocytosis ; Congenital dyserythropoietic anaemia type I ; Congenital dyserythropoietic anaemia type II ; Congenital dyserythropoietic anaemia type III; Delta Beta-thalassaemia ; Diamond- Blackfan-Anemia ; DMT1- deficiency anaemia ; Fanconi anaemia ; Gamma-glutamyl-cysteine synthetase deficiency ; GLRX5-related Sideroblastic anaemia ; Glucose phosphate isomerase deficiency ; Glucose-6-phosphate dehydrogenase deficiency ; Glutathione reductase deficiency ; Glutathione synthetase deficiency ; Haemoglobin C disease ; Haemoglobin D disease ; Haemoglobin E disease ; Haemoglobin H disease ; Haemoglobin Lepore ; Haemoglobin M with anaemia; Hereditary Elliptocytosis ; Hereditary persistance of fetal haemoglobin ; Hereditary Spherocytosis ; Hereditary Stomatocytosis ; Hexokinase deficiency ; Imerslund-Gräsbeck-Syndrome ; Iron - refractory iron deficiency Anemia ; Kearns-Sayre syndrome ; Lecithin cholesterol acyltransferase deficiency ; Other thalassaemias ; Other types of congenitale dyserythropoetic Anemia ; Paroxysmal nocturnal hemoglobinuria ; Pearson's Syndrome ; Phosphofructokinase deficiency ; Phosphoglycerate kinase deficiency ; Pyrimidine 5 nucleotidase deficiency ; Pyruvate kinase deficiency ; Refractory Anaemia (Myelodysplasia) ; Severe congenital hypochromic anemia with ringed sideroblasts ; Sickle cell anaemia ; Sickle cell trait; Sideroblastic anaemia associated with ataxia ; SLC25A38-related Sideroblastic anaemia ; Thiamine-responsive megaloblastic anemia; Triose phosphate isomerase deficiency ; Unstable haemoglobin ;and X linked sideroblastic anaemia (gene ALAS2).

Said RBC disorder can also be a conditions associated with stress erythropoiesis.

More preferably, said anaemia is selected from the group consisting of: Hereditary Spherocytosis; and Hereditary Xerocytosis.

The invention also provides a method of identifying a subject likely to have a mutation associated with a red blood cell (RBC) disorder comprising the steps of: (a) providing a sample of red blood cells previously obtained from a subject; (b) measuring the greatest projected area of each RBC in a population of said RBCs, wherein the greatest projected area of each RBC is measured by:

(i) allowing said RBCs to sediment onto a surface; and

(ii) measuring the projected surface area of each RBC in a direction substantially normal to the plane of said surface;

(c) creating a projected area distribution of the RBCs from said measurements; (d) providing a database storing like projected area distributions of RBCs obtained from subjects including those with a range of such mutations; and (e) comparing said measured projected area distribution to those stored in said database to find a closest match, the potential mutation being that associated with the matched distribution.

Preferably, said mutation is associated with a gene selected from the group consisting of: ANK1 (coding for erythrocytic Ankyrin 1 protein); SPTB (coding for erythrocytic spectrin beta chain protein); SCL4A1 (coding for erythrocytic spectrin alpha chain protein); and EPB42 (coding for erythrocyte membrane protein band 4.2).

Also in any such method, it is preferred that said sample of red blood cells is diluted before measurement, preferably with a diluent containing an albumin.

Also in any such method, it is preferred that said cells are exposed to a stressor before measurement.

Preferably, said stressor is selected from the group consisting of: osmotic stress; pH stress; temperature stress; and mechanical stress.

In any method of the invention it is also preferred that said projected area is measured by superimposing an ellipse over an image of each RBC, said ellipse having major and minor radii $r_1$ and $r_2$, and said projected area being measured as $\pi \cdot r_1 \cdot r_2$.

Also in any such method using a database, it is preferred that said database of like projected area distributions contains averaged projected area distributions taken from a plurality of subjects.

The invention further provides apparatus for carrying out a method according to a method described herein, said apparatus comprising: (a) a sedimentation chamber for accepting a sample of RBCs; (b) imaging apparatus configured to image cells within said sedimentation chamber in a direction substantially normal to the plane of a surface of said sedimentation chamber once said RBCs have sedimented onto said surface; (c) a processor configured to measure the distribution of greatest projected areas of said cells; (d) a database storing projected area distributions of RBCs obtained from subjects including those with a range of disorders affecting RBC morphology; and (e) a processor configured to compare said measured distribution of projected areas to those stored in said database to find a closest match between said measured and stored projected area distributions.

Preferably, said sedimentation chamber comprises a microfluidic chamber.

Along with raw diagnosis, these methods and apparatus may be used for assessment of disease severity for individual patients. Furthermore they may also be used for monitoring of therapeutic efficacy of treatment comparing the changes in projected areas distribution density for individual patient over time.

Among the advantages of the inventions is that the techniques exclude handling- and dryingrelated deformations of morphology and allows visualisation of shapes even for cells with unstable membrane such as those for rare hereditary hemolytic anemia.

Brief Description of the Figures

The invention will be described with reference to the accompanying drawings, in which:

Figure 1 is an average population density distribution of the projected areas of samples of red blood cells taken from healthy subjects;

Figure 2 illustrates a method of approximating the projected area of a red blood cell;

Figure 3 shows area population density distributions of RBCs from subjects diagnosed with a Band 3 mutation;

Figure 4 shows the mean area population density distributions of RBCs from subjects diagnosed with a Band 3 mutation compared to that of healthy volunteers;

Figure 5 shows area population density distributions of RBCs from subjects diagnosed with an SPTA mutation;

Figure 6 shows the mean area population density distributions of RBCs from subjects diagnosed with an SPTA mutation compared to that of healthy volunteers;

Figure 7 shows area population density distributions of RBCs from subjects diagnosed with an SPTB mutation;

Figure 8 shows the mean area population density distributions of RBCs from subjects diagnosed with as SPTB mutation compared to that of healthy volunteers;

Figure 9 shows mean area population density distributions of RBCs from subjects diagnosed with a range of pathologies and from a healthy control subject;

Figure 10 shows a flow diagram of a method of the invention; and

Figures 11-14 illustrate, schematically, apparatus for carrying out methods of the invention.

Description of Preferred Embodiments

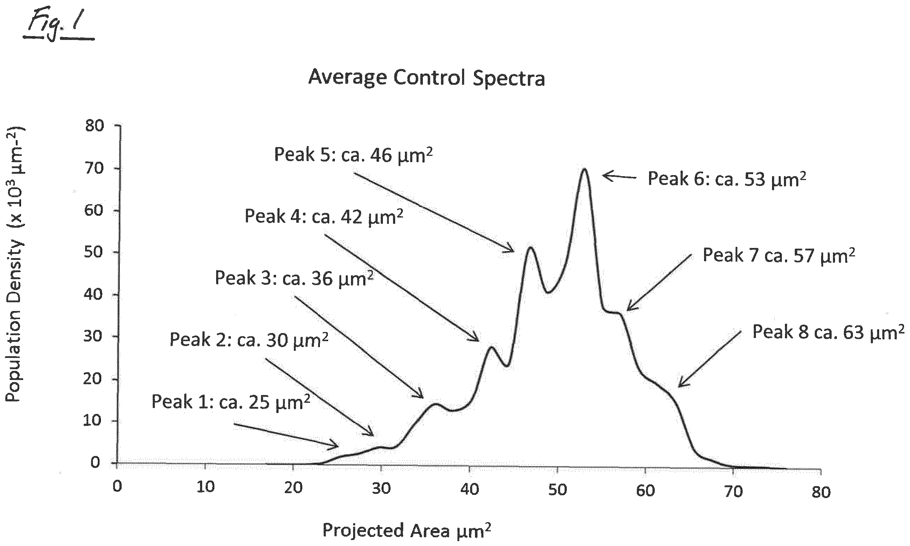

The inventors have surprisingly found that when the projected surface areas of members of a population of red blood cells are measured, the areas do not form a smooth distribution, but are clustered around characteristic peaks. These distributions resemble a projected area spectrum, characteristic of the state of the blood sample and reflect several morphologically defined populations of RBCs corresponding to well-known classes of cells (e.g. discocytes, disco-stomatocytes, stomatocytes, spherocytes, echinocytes). Figure 1 shows the average projected surface area distribution (or "spectrum") from eleven samples of blood from healthy subjects. Heparinized blood samples were suspended in a dilution medium at a dilution of 1:1000. The cells were allowed to sediment on a microscope slide, and were imaged using a Zeiss Axiovert 200M microscope with a 100x objective. When the cells sediment, they lie with their largest faces substantially parallel to the plane of the slide (rather than e.g. landing on their edges), and so the image observed is in effect the largest projected surface area of the RBC.

In this experiment, the dilution medium was a blood plasma analogue of the following composition:

| Component | Concentration (mM) |
|---|---|
| NaCl | 140 |
| KCl | 4 |
| $MgCl_2$ | 0.75 |
| CaCl2 | 2 |
| ZnCl2 | 0.015 |
| alanine | 0.2 |
| glutamate-Na | 0.2 |
| glycine | 0.2 |
| arginine | 0.1 |
| glutamine | 0.6 |
| glucose | 10 |
| HEPES-imidazole | 20 |

In addition, the medium contained 0.1% (w/v) BSA (bovine serum albumin), and was adjusted to pH 7.4. The procedure was carried out at room temperature (22-27°C, typically 25°C).

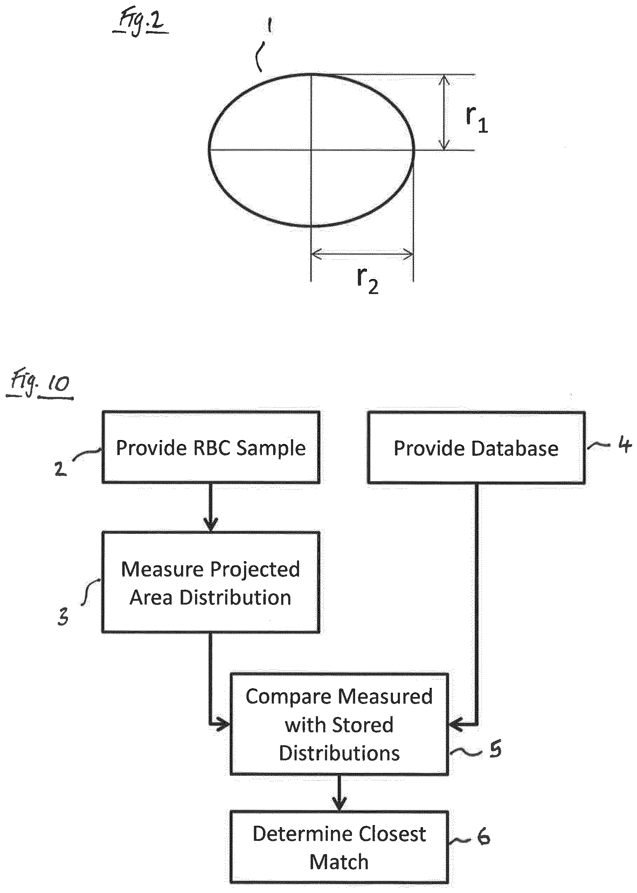

The projected cell areas were measured by image analysis, approximating the cell borders as an ellipse having major and minor radii $r_1$ and $r_2$, and the projected area being measured as $\pi . r_1 . r_2$. Figure 2 illustrates this measurement showing the ellipse 1 and the major and minor radii $r_1$ and $r_2$.

Returning to Figure 1, eight characteristic peaks can be seen, each one centred approximately around the projected cell areas indicated. The distribution of projected areas is presented as a probability distribution, the area under the curve between any two projected areas being the probability of finding a cell within that area range.

The inventors were able to associate each of these peaks with a particular cell morphology, by visual inspection. The peaks corresponded to morphological forms as follows:

Peak 1: terminal echinocytosis
Peak 2: echinocytosis
Peak 3: stomato-RBC
Peak 4: stomato-biconcave
Peak 5: tense biconcave
Peak 6: common biconcave
Peak 7: wavy relaxed biconcave
Peak 8: relaxed biconcave

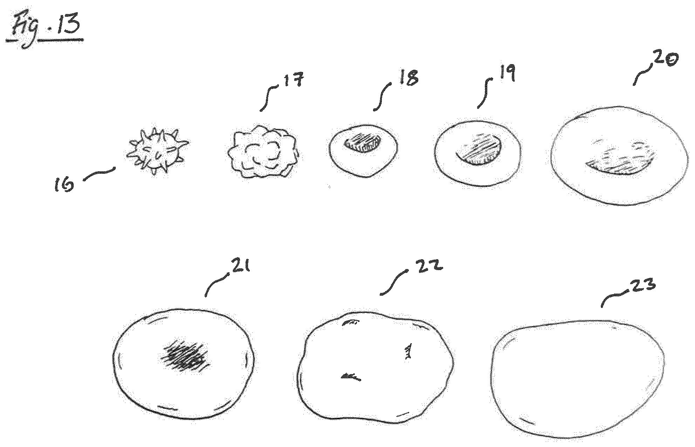

The morphological forms of these cells are illustrated in Figure 13, showing terminal echinocytosis 16, echinocytosis 17, stomato-RBC 18, stomato-biconcave 19, tense biconcave 20, common biconcave 21, wavy relaxed biconcave 22 and relaxed biconcave 23 cells.

The inventors obtained blood samples from subjects that had been diagnosed with a range of rare anaemias, and performed the same analysis.

Figure 3 shows the projected area distributions of RBCs from four subjects (identified as P005, P007, P053 and P059) diagnosed as having a mutation in the gene coding for the Band 3 anion exchanger protein (also known as anion exchanger 1[AE1], band 3, or solute carrier family 4 member 1 [SLC4A1]). It can be seen that, although there is some intra-subject variability, each of the morphological spectra (i.e. the area distributions) show very similar characteristic features.

Figure 4 shows the average projected area density distributions of RBCs for subjects having the Band 3 mutation vs. average plot for 11 healthy control subjects. The differences in the spectra are evident, for example, and most notably, the difference in relative sizes of peaks 5 and 6 (located at 46 $\mu m^2$ and 53 respectively).

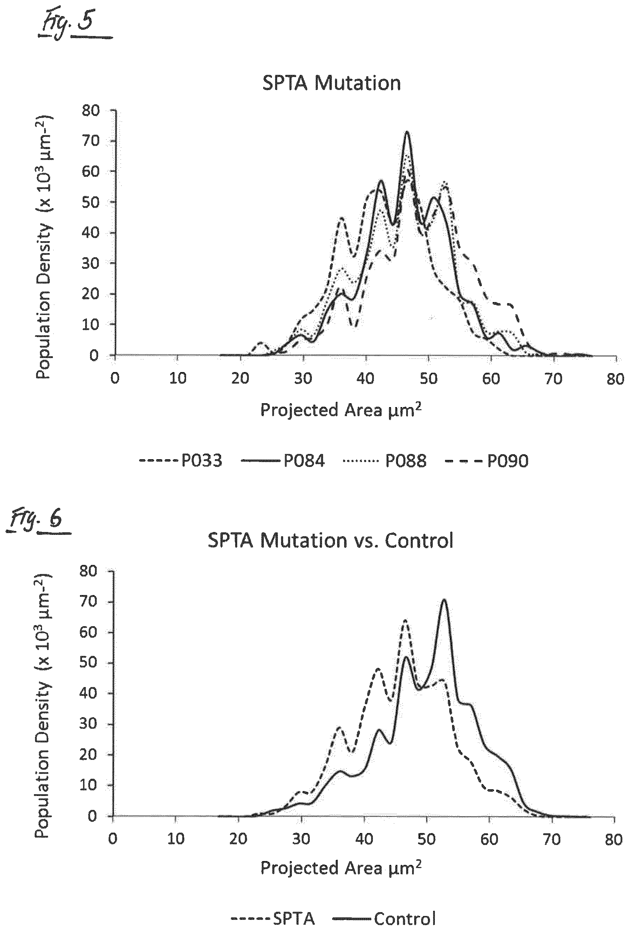

Figure 5 shows the projected area distributions of RBCs from four subjects (identified as P033, P084, P088, and P090) diagnosed as having a mutation in the SPTA gene, coding for erythrocytic spectrin alpha chain protein, causing blood disorders including spherocytic haemolytic anemia (hereditary spherocytosis)). Again, although there is some intra-subject variability, each of the morphological spectra (i.e. the area distributions) show very similar characteristic features.

Figure 6 shows the average projected area distributions of red blood cells for subjects having the SPTA mutation vs. healthy controls. Characteristic differences include a decrease in the relative abundance of cells having larger projected areas and a marked reduction in the proportion of cells in peak 6, located around 53 $\mu m^2$.

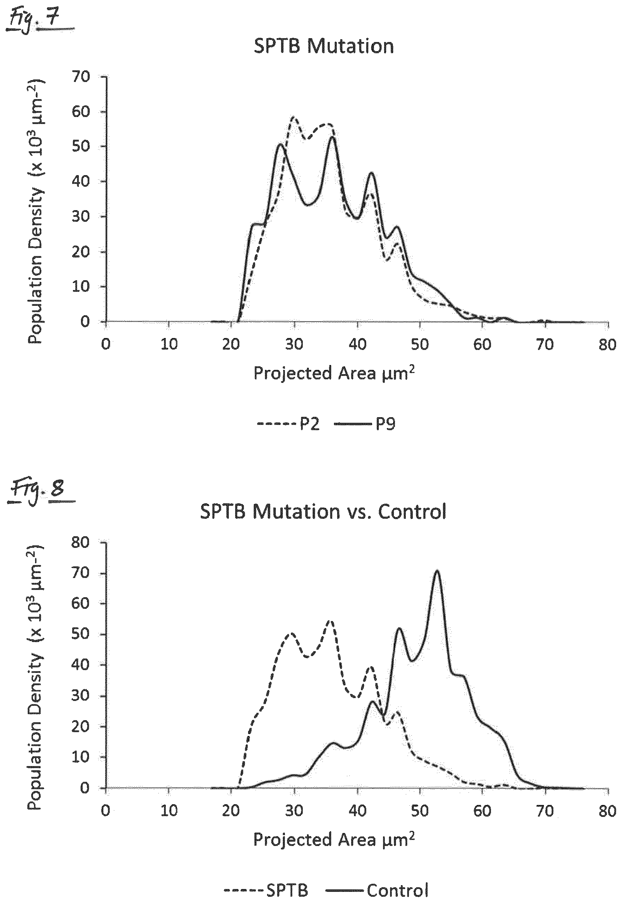

Figure 7 shows the projected area distributions of RBCs from two subjects (identified as P2 and P9) diagnosed as having a mutation in the SPTB gene, coding for erythrocytic spectrin beta chain protein. Again, although some variability is present the two morphological spectra show similar characteristics.

Figure 8 shows the average projected area distributions of red blood cells for subjects having the SPTB mutation vs. healthy controls. Marked characteristic differences include a much higher presence of smaller projected area cells in the SPTB subjects compared to the controls.

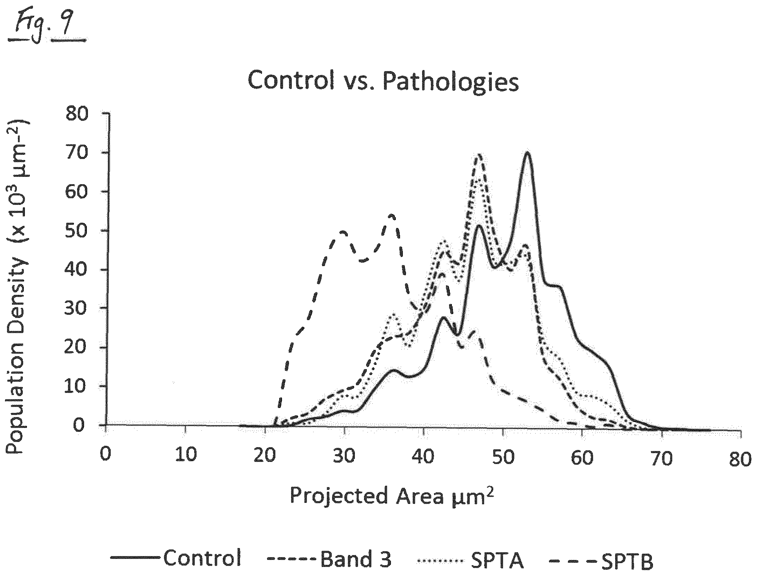

Figure 9 compares the projected area spectra for the three above pathologies vs. spectra from healthy control subjects.

The differences in these projected area spectrum therefor provide a method of differentiating between the different pathologies, or at least pre-screening subjects to select those who might benefit from a more detailed diagnostic study such as genetic sequencing.

A variety of heuristics may be used to assign a putative pathology to a blood sample. Figure 10 illustrates, as a flow diagram, a method for pre-screening subjects suspected of having an RBC disorder, or diagnosing such a disorder.

A sample of RBCs is provided 2, previously taken from a subject. A projected area distribution of the RBCs is measured 3. In order to take the measurement, the sample is preferably diluted. If sedimentation onto a surface is to be used to determine the maximum projected surface area, the sample is preferably diluted by approximately a factor of 1:1000, but this dilution could also by 1:5000, 1:2000, 1:500 or even 1:200.

The dilution medium should be selected so as not to change the morphological spectrum of the RBCs prior to measurement. The inventors have found that this may be achieved by using a medium that mimics blood plasma. In this regard, it is convenient to use a substitute such as the dilution medium described above. Alternative isotonic media should contain an albumin, preferably an albumin such as BSA (bovine serum albumin) or FCS (foetal calf serum). For example, PBS (phosphate buffered saline) or 0.9% NaCl solution each supplemented with 0.1% albumin, such as BSA would also be suitable.

The maximum projected area of each RBC is conveniently measured by allowing the cells to sediment onto a surface, thereby orienting themselves with their largest surface area parallel to the plane of that surface. Allowing the cells to sediment before imaging is, however, a simple and preferred method of achieving the projected surface area distribution. Such a method also aids in focussing the imaging apparatus, as all of the cells are essentially lying in the same plane.

It will also be appreciated that, although the present analysis is presented using a continuous distribution of projected cell areas, a similar analysis may be carried out by assigning each cell to a "bin" depending on its projected area. For example, by knowing the position of the peaks in the distribution, bins for each peak (and therefore each RBC morphology) may be constructed e.g. as follows:

| Bin | Peak Position ($\mu m^2$) | Lower Bound ($\mu m^2$) | Upper Bound ($\mu m^2$) | Morphology |
|---|---|---|---|---|
| 1 | 25 | 20* | 27.5 | terminal echinocytosis |
| 2 | 30 | 27.5 | 33 | echinocytosis |
| 3 | 36 | 33 | 39 | stomato-RBC |
| 4 | 42 | 39 | 44 | stomato-biconcave |
| 5 | 46 | 44 | 49.5 | tense biconcave |

(continued)

| Bin | Peak Position ($\mu$m$^2$) | Lower Bound ($\mu$m$^2$) | Upper Bound ($\mu$m$^2$) | Morphology |
|---|---|---|---|---|
| 6 | 53 | 49.5 | 55 | common biconcave |
| 7 | 57 | 55 | 60 | wavy relaxed biconcave |
| 8 | 63 | 60 | 65* | relaxed biconcave |

*The choice of these values may be chosen to exclude artefacts from imaging cell fragments, or platelets (for the lower bound), or larger cells such as white blood cells (for the upper bound).

A database 4 is also provided having representative RBC area distributions (either continuous or in bins, e.g. as above) taken from subjects with known pathologies, as well as healthy controls.

The measured projected area distribution of RBCs from the sample can then be compared 5 with those stored in the database to find a closest match 6.

Various methods of matching the measured distribution to the stored distributions will be evident to the skilled person. For example, among non-linear methods, a neural network may be trained to identify a measured peak as being most representative of a particular pathology. Similarly, genetic algorithms could also be used to good effect. A linear approach could also be taken, such as the use of cross-correlation between measured and stored spectra, or a simple approach such as singular value decomposition may be used.

In order to demonstrate this, on the relatively small dataset obtained by the inventors thus far, a singular value decomposition analysis using a least squares approach was applied to the data.

Mean values for the projected area distributions for each of the pathologies and for control subjects were calculated. These data are those presented graphically in Figure 9. These correspond to the database 4 of projected area number distributions.

Each of the patient and control samples was then compared against the database and a least squares difference calculated between each sample and the reference distributions. The least square difference (LSD) was calculated as follows:

$$LSD_j = \sum_{1}^{n} \left(S_i - R_{i,j}\right)^2$$

Where:

$LSD_j$ is the least square difference for reference distribution $j$;
$S_i$ is the population density of the sample at projected area $i$;
$R_{i,j}$ is the population density of the reference distributions at projected area $i$ for reference distribution $j$; and where the continuous distribution is approximated by $n$ data points.

The reference distribution having the lowest LSD gives an indication of the pathology represented by the sample. Table 1 shows the results of this analysis

**Table 1 - Determination of Closest Match**

| | LSD compared to reference distributions | | | | RBC Pathology | | | |
|---|---|---|---|---|---|---|---|---|
| Sample | **Band 3** | **SPTA** | **SPTB** | **Normal** | **Actual** | **Prediction** | **Correct** | **% Correct** |
| P005 | 327 | 840 | 15333 | 4972 | Band 3 | Band 3 | YES | |
| P007 | 269 | 416 | 13563 | 4350 | Band 3 | Band 3 | YES | |
| P053 | 360 | 607 | 11415 | 5401 | Band 3 | Band 3 | YES | |
| P059 | 692 | 1141 | 15539 | 3119 | Band 3 | Band 3 | YES | 100% |
| P033 | 2014 | 1800 | 9803 | 8907 | SPTA | SPTA | YES | |
| P084 | 513 | 412 | 16201 | 4216 | SPTA | SPTA | YES | |
| P088 | 428 | 230 | 14219 | 2788 | SPTA | SPTA | YES | |
| P090 | 2124 | 1281 | 17602 | 714 | SPTA | Normal | NO | 75% |
| P2 | 15794 | 15745 | 375 | 22758 | SPTB | SPTB | YES | |

(continued)

| | LSD compared to reference distributions | | | | RBC Pathology | | | |
|---|---|---|---|---|---|---|---|---|
| Sample | Band 3 | SPTA | SPTB | Normal | Actual | Prediction | Correct | % Correct |
| P9 | 12056 | 12056 | 375 | 18939 | SPTB | SPTB | YES | 100% |
| C006 | 12075 | 10131 | 26187 | 2800 | Normal | Normal | YES | |
| C008 | 664 | 675 | 17358 | 2693 | Normal | Band 3 | NO | |
| C034 | 8064 | 6699 | 25335 | 819 | Normal | Normal | YES | |
| C054 | 3549 | 3108 | 22057 | 489 | Normal | Normal | YES | |
| C060 | 12751 | 11380 | 30132 | 2900 | Normal | Normal | YES | |
| C062 | 6268 | 5687 | 28023 | 1104 | Normal | Normal | YES | |
| C085 | 10394 | 8924 | 26628 | 1666 | Normal | Normal | YES | |
| C089 | 3829 | 3264 | 23637 | 519 | Normal | Normal | YES | |
| C091 | 518 | 305 | 12664 | 3592 | Normal | SPTA | NO | |
| C | 5640 | 4609 | 20359 | 549 | Normal | Normal | YES | |
| C2 | 725 | 641 | 12772 | 2810 | Normal | SPTA | NO | 73% |

It can be seen that, even with the small amount of data used in this analysis, and with a simple least squares discriminator, the technique correctly identified the pathology associated with a sample for over 80% of all samples. It is expected that with more data, and a more sophisticated matching algorithm, better predictive power will ensue.

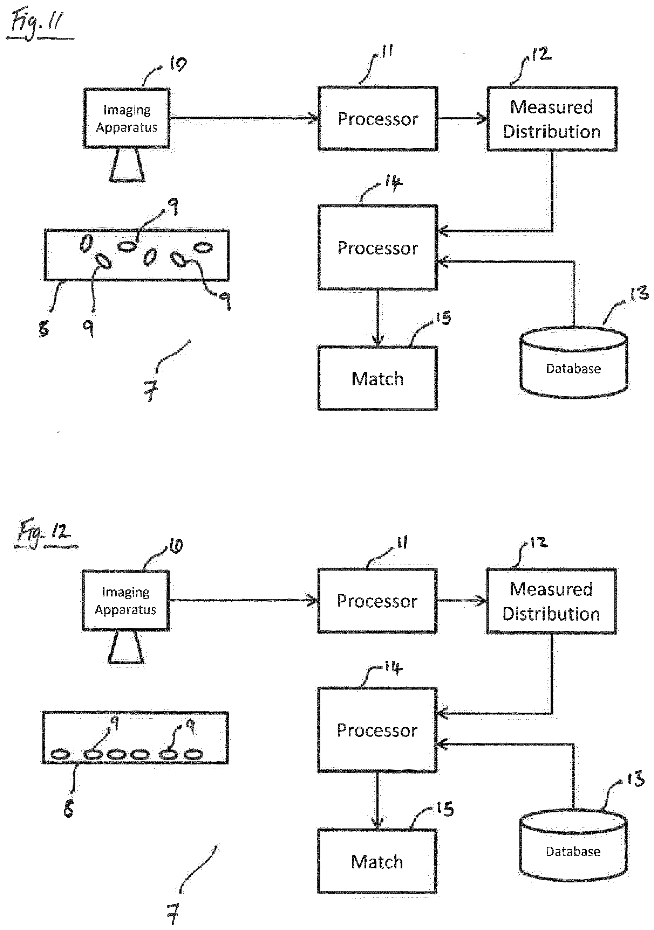

Figure 11 illustrates, schematically, an embodiment of an apparatus for carrying out a method of pre-screening, diagnosis or patient monitoring as described herein, generally indicated by 7. The apparatus comprises a chamber 8 for accepting and holding a sample of red blood cells 9. In this embodiment, the cells 9 are suspended within the fluid in the chamber. They may be allowed to sediment, into a configuration illustrated schematically in Figure 12 where they lie on a bottom surface of the chamber 8. Imaging apparatus 10 is also provided, e.g. in the form of a camera and microscope arrangement that can pass images to a processor 11 to produce a measured distribution 12. A database 13 is also provided, having projected cell area distributions from known pathologies and healthy subjects. The same 11 or a different processor 14 is also configured to compare the measured distribution 12 with those stored in the database 13, in order to find a closest match 15, and thereby indicate a putative assessment of the blood sample, e.g. by producing a diagnosis, or a pre-screening indicator.

Typically around 2000 RBCs are measured by automatic image analysis, but smaller numbers could also be counted, say 200, 500, or 1000 RBCs. As the field of view of most imaging systems is not able to image such numbers, the apparatus is preferably arranged to allow the field of view of the imaging apparatus and chamber to move relative to one another so that sufficient numbers of cells may be measured. In particularly preferred embodiments, a microfluidic cell may be employed, to allow samples to be moved into and out of an imaging chamber, which itself may be long enough to allow sufficient cells to sediment on its base such that they can be scanned with the imaging system.

Whilst this application has been described primarily in the context of identifying pathological conditions of red blood cells, the measurement of a morphological profile of a sample of red blood cells also has other applications.

Other diseases, such as diabetes, are known to affect the morphology of red blood cells in a subject, even though diabetes is not *per se* a pathology of RBCs. The techniques could also equally be used to identify disorders of RBC ion transport, and metabolic structure disorders of RBCs.

Furthermore, it is also known that as donated blood is stored, the morphological pattern of the red blood cells changes over time. The methods of measuring the morphological profile of a sample of red blood cells can therefore be applied to samples taken from stored, donated blood to assess potentially unwanted changes during storage.

The techniques described here could also be used to study osmotically induced red blood cell shrinkage or swelling and compensatory responses by the cells.

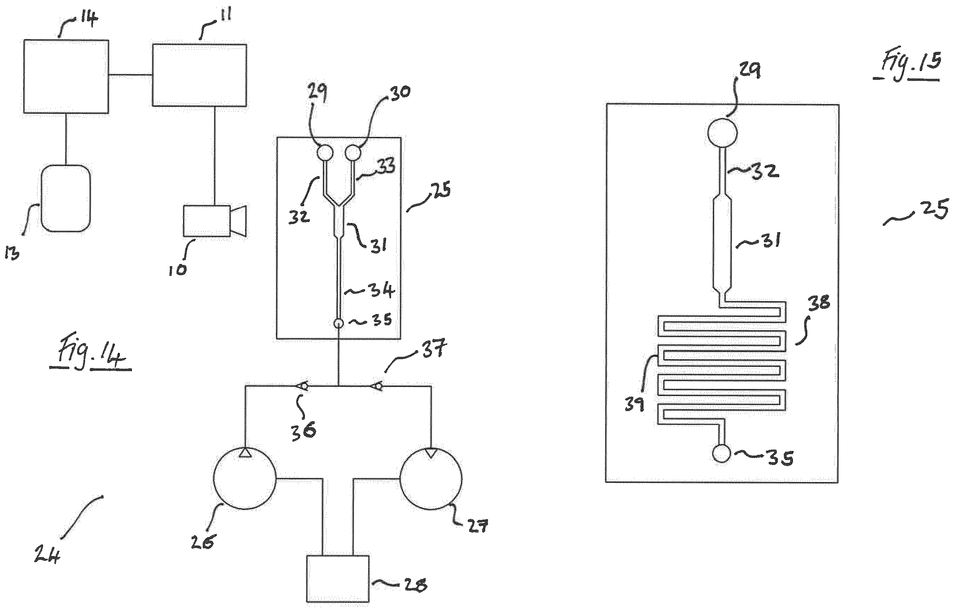

Figure 14 illustrates apparatus for carrying out a method of RBC analysis as described herein, generally indicated by 24. The microfluidic test apparatus 24 comprises a microfluidic device 25, first and second pumps 26 and 27, respectively, and a controller 28 that operates the pumps 26, 27.

The microfluidic device 25 comprises a first reservoir 29 and a second reservoir 30 for receiving a first fluid and a second fluid, respectively, and a microfluidic test region 31 that may serve as the sedimentation chamber for analysis of the RBCs. A first microfluidic pathway 32 is provided between the first reservoir 29 and the microfluidic test region 31. A second microfluidic pathway 33 is provided between the second reservoir 30 and the microfluidic test region 31. In the microfluidic device 25 illustrated in Fig. 14, a further microfluidic pathway 34 is provided between the microfluidic test region 31 and a port 35.

The first pump 26 is connected to the port 34 via a valve 36. The first pump 26 and valve 36 are arranged to pump a priming fluid into the port 34 when operated. The first pump 26 may be a syringe pump, in which the syringe filled with the priming fluid. The priming fluid may contain a wetting agent to reduce air being trapped in the microfluidic device 25.

The second pump 27 is connected to the port 35 via a valve 37. The second pump 27 and valve 37 are arranged to apply suction at the port 35 when operated and draw fluid therefrom. The valves 36 and 37 may take any suitable form, including a one-way valve, non-return valve, or an activated valve. In some embodiments the valves 36, 37 may be omitted.

The controller 28 is configured to control operation of the first and second pumps 26 and 27, and the valves 36, 376 where the valves are activated. The controller 28 may be any suitable device such as a microcontroller, embedded controller, programmable logic controller (PLC), microprocessor, portable computing device or computer and may include a control program. The controller 28 is configured to operate the first pump 26 to prime the microfluidic device 12. The first pump 14 preferably has a pump rate in the order of mL/second; this relatively high flow rate aids priming the microfluidic device 25 and reduces air entrapment. The controller 28 operates the first pump 26 to pump priming fluid into the microfluidic device 25 such that priming fluid enters the reservoirs 29, 30.

After priming, the first and second fluids are then added to the reservoirs 29 and 30, respectively. Where priming fluid has entered the reservoirs 29, 30, in some embodiments the priming fluid may be removed before the first and second fluids are added. The first fluid comprises a sample of red blood cells, which may be suitable diluted with a dilution medium as described above, typically at a dilution of approximately 1:1000. The second fluid is chosen according to the test requirements and may for example include a label and/or a stressor to the cells that cause a distinctive change in cells which may include cell lysis, aggregation, swelling, shrinkage, and/or shape change. In some embodiments, a series of second fluids may be added one by one to the second reservoir 30 as a test is performed, each second fluid having a different stressors, stressor concentration, and/or different labels.

If no stressor or other second reagent is required, the second reservoir 30 and its microfluidic passage 33 may be omitted.

The controller 28 is configured to operate the second pump 27 to draw a test volume of first fluid from the first reservoir 29 into the microfluidic test region 31. If used, a volume of second fluid will also be drawn from the second reservoir 30, according to the dimensions of the microfluidic pathways 32, 33. Since the second pump 27 applies suction to the port 35, pressure on the cells in the first fluid is limited. Using a pump to 'push' the first fluid through the microfluidic device 25 can result in higher pressure on the cells and cause cell ruptures, which may affect testing. It is preferred that the second pump 27 has a pump rate in the order of μL/second.

In the microfluidic device 25 shown in Fig. 14, the microfluidic test region 31 comprises a microfluidic channel into which the first and (if used) second fluids flow. Other forms of microfluidic test region 31 may be employed; for instance, the microfluidic test region 31 may comprise a microfluidic channel formed into a spiral. Forming the microfluidic test region 31 in a spiral may permit the imaging device 10 to capture fluid flow at several locations along the microfluidic test region 31 in a small area covered by a single image.

In order to perform an analysis as described herein a suitable protocol can include configuring the pump controller 28 to draw a sample of diluted RBCs into the microfluidic test region 31, to stop the flow to allow the RBCs to sediment in the microfluidic test region (i.e. to use it as a sedimentation chamber) and send a signal to the imaging system 10, 11 to cause it to capture an image of the sedimented cells. The pump controller can then restart the pump 27 to allow a further sample of diluted RBCs to be drawn into the microfluidic test region 31, and again stop the pump 27 and signal the imaging system 10, 11 to capture a further image of a second aliquot of diluted, sedimented RBCs. This can be repeated as often as required until a required number of cells have been imaged. The imaging system 10, 11 may be configured to send instructions to the pump controller 28 indicating whether sufficient images have been obtained.

It will be appreciated that the imaging apparatus 10 may be arranged to image the sedimented RBCs through either the top or bottom of the microfluidic device 25. Additionally, the microfluidic device 25 and imaging apparatus 10 may be configured such that the two are moved relative to each other after the cells have sedimented, to enable a larger field of view to be captured.

Figure 15 illustrates, schematically, an alternative microfluidic device 25 that may be used as part of the apparatus. This device 25 differs from that illustrated in Figure 14 in that a single reservoir 29 is provided, with a single microfluidic channel 32 connecting it to the microfluidic test region 31. This configuration is preferred where no second fluid (e.g. a stressor fluid or stain) is required in the methodology.

The device further comprises a microfluidic waste region 38 provided between the microfluidic test region 31 and the port 35. The microfluidic waste region 38 may comprise a circuitous microfluidic pathway 39. Whilst shown in two dimensions in the drawings for clarity it will be appreciated that the pathway 39 may be formed in three dimensions. The microfluidic waste region 38 defines a microfluidic volume commensurate with the test volume to prevent the test fluid from reaching the port 30. The microfluidic waste region 38 prevents the test fluid from leaving the microfluidic device 25, thereby avoiding crosscontamination that would result if some of the first or second fluids were to leave the microfluidic device 25 and then subsequently be pumped into another microfluidic device during the priming thereof. It will be appreciated that such a microfluidic waste region 28 may also be employed in an analogous fashion to the test device 25 of Figure 13.

**Claims**

1. A method of measuring the morphological profile of a sample of red blood cells (RBCs) comprising the steps of:

   (a) measuring (3) the greatest projected area of each RBC in a population of said RBCs, wherein the greatest projected area of each RBC is measured by:

   (i) allowing said RBCs to sediment onto a surface; and
   (ii) measuring the projected surface area of each RBC in a direction substantially normal to the plane of said surface;

   (b) creating a projected area distribution of the RBCs from said measurements; and
   (c) identifying characteristic peaks from said projected area distribution;

   each characteristic peak having an associated population density and corresponding with a projected area, the population densities of the characteristic peaks indicating the proportion of phenotypic cell types within the RBC sample.

2. A method according to Claim 1 wherein said characteristic peaks are those peaks located closest to the following projected areas:

   Peak 1: 25 $\mu$m$^2$
   Peak 2: 30 $\mu$m$^2$
   Peak 3: 36 $\mu$m$^2$
   Peak 4: 42 $\mu$m$^2$
   Peak 5: 46 $\mu$m$^2$
   Peak 6: 53 $\mu$m$^2$
   Peak 7: 57 $\mu$m$^2$
   Peak 8: 63

   and wherein each peak indicates the following RBC morphology:

   Peak 1: terminal echinocytosis
   Peak 2: echinocytosis
   Peak 3: stomato-RBC
   Peak 4: stomato-biconcave
   Peak 5: tense biconcave
   Peak 6: common biconcave
   Peak 7: wavy relaxed biconcave
   Peak 8: relaxed biconcave.

3. A method of pre-screening for a disorder affecting red blood cells (RBCs) in a subject comprising the steps of:

   (a) providing (2) a sample of red blood cells previously obtained from a subject;
   (b) measuring (3) the greatest projected area of each RBC in a population of said RBCs, wherein the greatest projected area of each RBC is measured by:

   (i) allowing said RBCs to sediment onto a surface; and
   (ii) measuring the projected surface area of each RBC in a direction substantially normal to the plane of said surface;

   (c) creating a projected area distribution of the RBCs from said measurements;

(d) providing (4) a database storing like projected area distributions of RBCs obtained from subjects including those with a range of such disorders; and

(e) comparing (5) said measured projected area distribution to those stored in said database to find (6) a closest match, the potential RBC disorder being that associated with the matched distribution.

4. A method according to Claim 3 wherein said disorder is a red blood cell disorder.

5. A method according to Claim 4 wherein said disorder is an anaemia.

6. A method according to Claim 5 wherein said disorder is selected from the group consisting of:

Hereditary Spherocytosis; and
Hereditary Xerocytosis.

7. A method of identifying a subject likely to have a mutation associated with a red blood cell (RBC) disorder comprising the steps of:

(a) providing (2) a sample of red blood cells previously obtained from a subject;
(b) measuring (3) the greatest projected area of each RBC in a population of said RBCs, wherein the greatest projected area of each RBC is measured by:

(i) allowing said RBCs to sediment onto a surface; and
(ii) measuring the projected surface area of each RBC in a direction substantially normal to the plane of said surface;

(c) creating a projected area distribution of the RBCs from said measurements;
(d) providing (4) a database storing like projected area distributions of RBCs obtained from subjects including those with a range of such mutations; and
(e) comparing (5) said measured projected area distribution to those stored in said database to find (6) a closest match, the potential mutation being that associated with the matched distribution.

8. A method according to Claim 7 wherein said mutation is associated with a gene selected from the group consisting of:

ANK1 (coding for erythrocytic Ankyrin 1 protein);
SPTB (coding for erythrocytic spectrin beta chain protein);
SCL4A1 (coding for solute carrier family 4 member 1);
SPTA (coding for erythrocytic spectrin alpha chain protein); and
EPB42 (coding for erythrocyte membrane protein band 4.2).

9. A method according to any preceding claim where said sample (2) of red blood cells is diluted before measurement.

10. A method according to Claim 9 wherein said cells are diluted with a diluent containing an albumin.

11. A method according to any preceding claim wherein said cells are exposed to a stressor before measurement.

12. A method according to Claim 11 wherein said stressor is selected from the group consisting of:

osmotic stress;
pH stress;
temperature stress; and
mechanical stress.

13. A method according to any preceding claim wherein said projected area is measured by superimposing an ellipse over an image of each RBC, said ellipse having major and minor radii $r_1$ and $r_2$, and said projected area being measured as $\pi \cdot r_1 \cdot r_2$.

14. A method according to any of Claims 3 to 13 wherein said database of like projected area distributions contains averaged projected area distributions taken from a plurality of subjects.

**15.** Apparatus (7) for carrying out a method according to any preceding claim comprising:

(a) a sedimentation chamber (8) for accepting a sample of RBCs;
(b) imaging apparatus (10) configured to image cells (9) within said sedimentation chamber (8) in a direction substantially normal to the plane of a surface of said sedimentation chamber (8) once said RBCs have sedimented onto said surface;
(c) a processor (11) configured to measure the projected surface area of each RBC in a direction substantially normal to the plane of said surface and to measure the distribution (12) of greatest projected areas of said cells (9);
(d) a database (13) storing projected area distributions of RBCs obtained from subjects including those with a range of disorders affecting RBC morphology;
(e) a processor (14) configured to compare said measured distribution (12) of projected areas to those stored in said database (13) to find a closest match (15) between said measured and stored projected area distributions.

## Patentansprüche

**1.** Verfahren zum Messen des morphologischen Profils einer Probe von roten Blutkörperchen (RBK), umfassend die Schritte:

(a) Messen (3) der größten projizierten Fläche jedes RBK in einer Population der RBK, wobei die größte projizierte Fläche jedes RBK gemessen wird durch:

(i) Absetzenlassen der RBK auf einer Oberfläche; und
(ii) Messen des projizierten Oberflächenbereichs jedes RBK in einer Richtung, die im Wesentlichen senkrecht zu der Ebene der Oberfläche ist;

(b) Erzeugen einer Verteilung der RBK auf einer projizierten Fläche aus den Messungen; und
(c) Identifizieren von charakteristischen Peaks aus der Verteilung auf der projizierten Fläche;

wobei jeder charakteristische Peak eine assoziierte Populationsdichte aufweist und einer projizierten Fläche entspricht, wobei die Populationsdichten der charakteristischen Peaks den Anteil von phänotypischen Zelltypen innerhalb der RBK-Probe angeben.

**2.** Verfahren nach Anspruch 1, wobei die charakteristischen Peaks jene Peaks sind, die sich am nächsten zu den folgenden projizierten Flächen befinden:

Peak 1: 25 $\mu m^2$
Peak 2: 30 $\mu m^2$
Peak 3: 36 $\mu m^2$
Peak 4: 42 $\mu m^2$
Peak 5: 46 $\mu m^2$
Peak 6: 53 $\mu m^2$
Peak 7: 57 $\mu m^2$
Peak 8: 63 $\mu m^2$,
und wobei jeder Peak die folgende RBK-Morphologie angibt:

Peak 1: Echinozytose im Endstadium
Peak 2: Echinozytose
Peak 3: Stomato-RBK
Peak 4: Stomato-bikonkav
Peak 5: Gespannt bikonkav
Peak 6: Normal bikonkav
Peak 7: Wellig, entspannt bikonkav
Peak 8: Entspannt bikonkav.

**3.** Verfahren zum Vorscreenen auf eine Erkrankung, die sich auf rote Blutkörperchen (RBK) auswirkt, bei einem Probanden, umfassend die Schritte:

(a) Bereitstellen (2) einer Probe von roten Blutkörperchen, die zuvor von einem Probanden bezogen wurde;
(b) Messen (3) der größten projizierten Fläche jedes RBK in einer Population der RBK, wobei die größte projizierte Fläche jedes RBK gemessen wird durch:

(i) Absetzenlassen der RBK auf einer Oberfläche; und
(ii) Messen des projizierten Oberflächenbereichs jedes RBK in einer Richtung, die im Wesentlichen senkrecht zu der Ebene der Oberfläche ist;

(c) Erzeugen einer Verteilung der RBK auf einer projizierten Fläche aus den Messungen;
(d) Bereitstellen (4) einer Datenbank, die ähnliche Verteilungen von RBK auf projizierten Flächen speichert, die von Probanden bezogen wurden, einschließlich jenen mit einer Reihe von derartigen Erkrankungen; und
(e) Vergleichen (5) der gemessenen Verteilung auf der projizierten Fläche mit jenen, die in der Datenbank gespeichert sind, um eine größte Übereinstimmung zu finden (6), wobei die potenzielle RBK-Erkrankung jene ist, die mit der übereinstimmenden Verteilung assoziiert ist.

4. Verfahren nach Anspruch 3, wobei die Erkrankung eine Erkrankung der roten Blutkörperchen ist.

5. Verfahren nach Anspruch 4, wobei die Erkrankung eine Anämie ist.

6. Verfahren nach Anspruch 5, wobei die Erkrankung ausgewählt ist aus der Gruppe bestehend aus:

Hereditäre Sphärozytose; und
hereditäre Xerozytose.

7. Verfahren zum Identifizieren eines Probanden, der wahrscheinlich eine Mutation aufweist, die mit einer Erkrankung der roten Blutkörperchen (RBK) assoziiert ist, umfassend die Schritte:

(a) Bereitstellen (2) einer Probe von roten Blutkörperchen, die zuvor von einem Probanden bezogen wurde;
(b) Messen (3) der größten projizierten Fläche jedes RBK in einer Population der RBK, wobei die größte projizierte Fläche jedes RBK gemessen wird durch:

(i) Absetzenlassen der RBK auf einer Oberfläche; und
(ii) Messen des projizierten Oberflächenbereichs jedes RBK in einer Richtung, die im Wesentlichen senkrecht zu der Ebene der Oberfläche ist;

(c) Erzeugen einer Verteilung der RBK auf einer projizierten Fläche aus den Messungen;
(d) Bereitstellen (4) einer Datenbank, die ähnliche Verteilungen von RBK auf projizierten Flächen speichert, die von Probanden bezogen wurden, einschließlich jenen mit einer Reihe von derartigen Mutationen; und
(e) Vergleichen (5) der gemessenen Verteilung auf der projizierten Fläche mit jenen, die in der Datenbank gespeichert sind, um eine größte Übereinstimmung zu finden (6), wobei die potenzielle Mutation jene ist, die mit der übereinstimmenden Verteilung assoziiert ist.

8. Verfahren nach Anspruch 7, wobei die Mutation mit einem Gen assoziiert ist, das ausgewählt ist aus der Gruppe bestehend aus:

ANK1 (für erythrozytäres Ankyrin-1-Protein codierend);
SPTB (für erythrozytäres Spektrin-Beta-Kettenprotein codierend);
SCL4A1 (für Mitglied 1 der Solute-Carrier-Familie 4 codierend);
SPTA (für erythrozytäres Spektrin-Alpha-Kettenprotein codierend); und
EPB42 (für Erythrozyten-Membranprotein, Bande 4.2, codierend).

9. Verfahren nach einem vorhergehenden Anspruch, wobei die Probe (2) von roten Blutkörperchen vor der Messung verdünnt wird.

10. Verfahren nach Anspruch 9, wobei die Zellen mit einem Verdünnungsmittel verdünnt werden, das ein Albumin enthält.

11. Verfahren nach einem vorhergehenden Anspruch, wobei die Zellen vor der Messung einem stressor ausgesetzt werden.

12. Verfahren nach Anspruch 11, wobei der stressor ausgewählt ist aus der Gruppe bestehend aus:

> osmotischer Belastung;
> pH-Wert-Belastung;
> Temperaturbelastung; und
> mechanischer Belastung.

13. Verfahren nach einem vorhergehenden Anspruch, wobei die projizierte Fläche durch Überlagern einer Ellipse auf einem Bild jedes RBK gemessen wird, wobei die Ellipse einen Haupt- und einen Nebenradius $r_1$ und $r_2$ aufweist und die projizierte Fläche als $\pi \cdot r_1 \cdot r_2$ gemessen wird.

14. Verfahren nach einem der Ansprüche 3 bis 13, wobei die Datenbank von ähnlichen Verteilungen auf projizierten Flächen gemittelte Verteilungen auf projizierten Flächen enthalten, die von mehreren Probanden genommen wurden.

15. Vorrichtung (7) zum Durchführen eines Verfahrens nach einem vorhergehenden Anspruch, umfassend:

> (a) eine Absetzkammer (8) zum Aufnehmen einer Probe von RBK;
> (b) eine Abbildungsvorrichtung (10), die dazu konfiguriert ist, Zellen (9) innerhalb der Absetzkammer (8) in einer Richtung, die im Wesentlichen senkrecht zu der Ebene einer Oberfläche der Absetzkammer (8) ist, abzubilden, sobald die RBK sich auf der Oberfläche abgesetzt haben;
> (c) einen Prozessor (11), der dazu konfiguriert ist, den projizierten Oberflächenbereich jedes RBK in einer Richtung, die im Wesentlichen senkrecht zu der Ebene der Oberfläche ist, zu messen und die Verteilung (12) von größten projizierten Flächen der Zellen (9) zu messen;
> (d) eine Datenbank (13), die Verteilungen von RBK auf projizierten Flächen speichert, die von Probanden bezogen wurden, einschließlich jenen mit einer Reihe von Erkrankungen, die sich auf die RBK-Morphologie auswirken;
> (e) einen Prozessor (14), der dazu konfiguriert ist, die gemessene Verteilung (12) von projizierten Flächen mit jenen, die in der Datenbank (13) gespeichert sind, zu vergleichen, um eine größte Übereinstimmung (15) zwischen den gemessenen und den gespeicherten Verteilungen auf projizierten Flächen zu finden.

## Revendications

1. Procédé de mesure du profil morphologique d'un échantillon de globules rouges (RBC), comprenant les étapes suivantes :

> (a) mesure (3) de la surface projetée maximale de chaque RBC dans une population desdits RBC, dans laquelle la surface projetée maximale de chaque RBC est mesurée par

>> (i) l'opération consistant à laisser lesdits RBC sédimenter sur une surface ; et
>> (ii) la mesure de la surface projetée de chaque RBC dans une direction pratiquement normale au plan de ladite surface ;

> (b) création d'une distribution de surfaces projetées des RBC à partir desdites mesures ; et
> (c) identification de pics caractéristiques à partir de ladite distribution de surfaces projetées ;

chaque pic caractéristique ayant une densité de population associée et correspondant à une surface projetée, les densités de population des pics caractéristiques indiquant la proportion de types cellulaires phénotypiques dans l'échantillon de RBC.

2. Procédé selon la revendication 1, dans lequel lesdits pics caractéristiques sont les pics situés au plus près des surfaces projetées suivantes :

> Pic 1 : 25 $\mu m^2$
> Pic 2 : 30 $\mu m^2$
> Pic 3 : 36 $\mu m^2$
> Pic 4 : 42 $\mu m^2$
> Pic 5 : 46 $\mu m^2$

Pic 6 : 53 $\mu$m$^2$
Pic 7 : 57 $\mu$m$^2$
Pic 8 : 63 $\mu$m$^2$
et dans lequel chaque pic indique la morphologie de RBC suivante :

Pic 1 : échinocytose terminale
Pic 2 : échinocytose
Pic 3 : stomato-RBC
Pic 4 : stomato-biconcave
Pic 5 : biconcave serré
Pic 6 : biconcave commun
Pic 7 : biconcave relâché ondulé
Pic 8 : biconcave relâché.

3. Procédé de pré-dépistage d'un trouble affectant les globules rouges (RBC) chez un sujet, comprenant les étapes suivantes :

(a) obtention (2) d'un échantillon de globules rouges préalablement obtenus à partir d'un sujet ;
(b) mesure (3) de la surface projetée maximale de chaque RBC dans une population desdits RBC, dans laquelle la surface projetée maximale de chaque RBC est mesurée par :

(i) l'opération consistant à laisser lesdits RBC sédimenter sur une surface ; et
(ii) la mesure de la surface projetée de chaque RBC dans une direction pratiquement normale au plan de ladite surface ;

(c) création d'une distribution de surfaces projetées des RBC à partir desdites mesures ;
(d) obtention (4) d'une base de données stockant des distributions de surfaces projetées analogues de RBC obtenus à partir de sujets comprenant ceux ayant un éventail de tels troubles ; et
(e) comparaison (5) de ladite distribution de surfaces projetées mesurée à celles stockées dans ladite base de données pour trouver (6) la concordance la plus proche, le trouble potentiel des RBC étant celui associé à la distribution concordante.

4. Procédé selon la revendication 3, dans lequel ledit trouble est un trouble des globules rouges.

5. Procédé selon la revendication 4, dans lequel ledit trouble est une anémie.

6. Procédé selon la revendication 5, dans lequel ledit trouble est choisi dans le groupe constitué par :

une sphérocytose héréditaire ; et
une xérocytose héréditaire.

7. Procédé d'identification d'un sujet susceptible d'avoir une mutation associée à un trouble des globules rouges (RBC), comprenant les étapes suivantes :

(a) obtention (2) d'un échantillon de globules rouges préalablement obtenus à partir d'un sujet ;
(b) mesure (3) de la surface projetée maximale de chaque RBC dans une population desdits RBC, dans laquelle la surface projetée maximale de chaque RBC est mesurée par :

(i) l'opération consistant à laisser lesdits RBC sédimenter sur une surface ; et
(ii) la mesure de la surface projetée de chaque RBC dans une direction pratiquement normale au plan de ladite surface ;

(c) création d'une distribution de surfaces projetées des RBC à partir desdites mesures ;
(d) obtention (4) d'une base de données stockant des distributions de surfaces projetées analogues de RBC obtenus à partir de sujets comprenant ceux ayant un éventail de telles mutations ; et
(e) comparaison (5) de ladite distribution de surfaces projetées mesurée à celles stockées dans ladite base de données pour trouver (6) la concordance la plus proche, la mutation potentielle étant celle associée à la distribution concordante.

**8.** Procédé selon la revendication 7, dans lequel ladite mutation est associée à un gène choisi dans le groupe constitué par :

ANK1 (codant la protéine ankyrine 1 érythrocytaire) ;
SPTB (codant la protéine spectrine chaîne bêta érythrocytaire) ;
SCL4A1 (codant le membre 1 de la famille 4 des transporteurs de solutés) ;
SPTA (codant la protéine spectrine chaîne alpha érythrocytaire) ; et
EPB42 (codant la bande 4.2 de protéine de membrane érythrocytaire).

**9.** Procédé selon l'une quelconque des revendications précédentes, dans lequel ledit échantillon (2) de globules rouges est dilué avant la mesure.

**10.** Procédé selon la revendication 9, dans lequel lesdites cellules sont diluées avec un diluant contenant une albumine.

**11.** Procédé selon l'une quelconque des revendications précédentes, dans lequel lesdites cellules sont exposées à un agent stressant avant la mesure.

**12.** Procédé selon la revendication 11, dans lequel ledit agent stressant est choisi dans le groupe constitué par :

un stress osmotique ;
un stress de pH ;
un stress de température ; et
un stress mécanique.

**13.** Procédé selon l'une quelconque des revendications précédentes, dans lequel ladite surface projetée est mesurée par superposition d'une ellipse sur une image de chaque RBC, ladite ellipse ayant des rayons majeur et mineur $r_1$ et $r_2$, et ladite surface projetée étant mesurée sous la forme $\pi \cdot r_1 \cdot r_2$.

**14.** Procédé selon l'une quelconque des revendications 3 à 13, dans lequel ladite base de données de distributions de surfaces projetées analogues contient des distributions de surfaces projetées moyennées prises à partir d'une pluralité de sujets.

**15.** Dispositif (7) pour mettre en œuvre un procédé selon l'une quelconque des revendications précédentes, comprenant :

(a) une chambre de sédimentation (8) destinée à accepter un échantillon de RBC ;
(b) un dispositif d'imagerie (10) configuré pour mettre sous forme d'images des cellules (9) à l'intérieur de ladite chambre de sédimentation (8) dans une direction pratiquement normale au plan d'une surface de ladite chambre de sédimentation (8) une fois que lesdits RBC ont sédimenté sur ladite surface ;
(c) un processeur (11) configuré pour mesurer la surface projetée de chaque RBC dans une direction pratiquement normale au plan de ladite surface et pour mesurer la distribution (12) des surfaces projetées maximales desdites cellules (9) ;
(d) une base de données (13) stockant des distributions de surfaces projetées de RBC obtenus à partir de sujets comprenant ceux ayant un éventail de troubles affectant la morphologie des RBC ;
(e) un processeur (14) configuré pour comparer ladite distribution mesurée (12) de surfaces projetées à celles stockées dans ladite base de données (13) pour trouver la concordance la plus proche (15) entre lesdites distributions de surfaces projetées mesurées et stockées.

Fig. 1

Average Control Spectra

Fig.3

## Morphological Spectra - Band 3 Mutation

----- P005 ———— P007 ·········· P053 – – – P059

Fig.4

## Band 3 Mutation vs. Control

----- Band 3 ———— Control

Fig. 5

## SPTA Mutation

-----P033 ——P084 ········ P088 ---P090

Fig. 6

## SPTA Mutation vs. Control

-----SPTA ——Control

_Fig. 7_

## SPTB Mutation

----- P2 ——— P9

_Fig. 8_

## SPTB Mutation vs. Control

----- SPTB ——— Control

EP 3 673 254 B1

## Fig. 9

Control vs. Pathologies

Population Density ($\times 10^3$ $\mu m^{-2}$) vs. Projected Area $\mu m^2$

——Control  -----Band 3  ·········SPTA  - - -SPTB

_Fig. 2_

_Fig. 10_

Fig. 11

Fig. 12

Fig. 13

Fig. 14

Fig. 15

EP 3 673 254 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DEB, N. ; CHAKROBORTY, S.** A Noble Technique for Detecting Anemia through Classification of Red Blood Cells in a Blood Smear. *IEEE International Conference on Recent Advances and Innovations in Engineering (ICRAIE-2014), May 09-11, Jaipur, India*
- **AMIREAULT PASCAL et al.** 2343: Contribution of Imaging Flow Cytometry to Storage Lesion Assessment: Identification of a Sub-Population of Morphologically Altered Erythrocytes. *BLOOD; 57TH ANNUAL MEETING OF THE AMERICAN-SOCIETY-OF-HEMATOLOGY, AMERICAN SOCIETY OF HEMATOLOGY, US; ORLANDO, FL, USA,* 03 December 2015, vol. 126 (23), ISSN 0006-4971, 2343
- **INNOCENT SAFEUKUI et al.** Surface Area Loss and Increased Sphericity Account for the Splenic Entrapment of Subpopulations of Plasmodium falciparum Ring-Infected Erythrocytes. *PLOS ONE,* 01 March 2013, vol. 8 (3
- **JAFERZADEH KEYVAN et al.** Quantitative investigation of red blood cell three-dimensional geometric and chemical changes in the storage lesion using digital holographic microscopy. *INTERNATIONAL SOCIETY FOR OPTICAL ENGINEERING, SPIE, PO BOX 10 BELLINGHAM WA 98227-0010 USA,* vol. 20 (11
- **HOA V. PHAM et al.** Real Time Blood Testing Using Quantitative Phase Imaging. *PLOS ONE,* 06 February 2013, vol. 8 (2